# EUROPEAN PATENT SPECIFICATION

(11) **EP 1 594 471 B1**
(45) Date of publication and mention of the grant of the patent: **25.03.2009**
(21) Application number: 04712644.6
(22) Date of filing: 19.02.2004
(51) Int. Cl.: A61K 9/14, A61K 9/20, A61K 9/16, A61K 31/573

(54) **Process for manufacturing a dexamethasone-containing formulation for oral administration**
Prozess zur Herstellung einer Dexamethason enthaltenden Formulierung zur oralen Anwendung
Procédé de fabrication d'une formulation contenant de la dexaméthasone pour administration orale

(30) Priority: 20.02.2003 US 448174 P
(43) Date of publication of application: 16.11.2005
(73) Proprietor: Constant Research & Development Limited, Dublin 24 (IE)
(72) Inventor: RICE, Paul, William, Bimeda R. & D. Limited, Dublin 24 (IE); MCHARDY, Nicholas, County Wicklow (IE)
(74) Representative: O'Brien, John Augustine
(86) International application number: PCT/IE2004/000024
(87) International publication number: WO 2004/073685

(56) References cited:
- WO-A-98/22095
- WO-A-98/35701
- GB-A- 970 440
- US-A- 3 231 468

## Description

### Field of the Invention

This invention relates to a method for formulating the corticosteroid dexamethasone into a granulation.

### Background of the Invention

The manufacture of drug formulations for pharmaceutical use can be complex in preparing a single drug formulation or a combination of drugs in a formulation for optimum efficacy. Different drugs can have very different properties and require different handling methods.

Dexamethasone is a very potent corticosteroid; it has thirty times the anti-inflammatory potency of cortisone (The Pharmacological basis of Therapeutics, Ninth edition, Goodman and Gilman, page 1466). As a result, only a small amount of drug is needed in the oral dosage form (tablet) which is given to the patient. Generally, only 0.25 mg dexamethasone is present in a 300 mg tablet (0.083%). The challenge is to uniformly disperse the small amount of drug in the tablet granulation to provide uniformity of dose in the compressed tablets.

Historically, dexamethasone has been incorporated into the tablet granulation either by direct compression or wet granulation.

Direct compression techniques use ordered mixing to gradually mix the dexamethasone with increasing amounts of excipient. While a uniform mix may result in the blender, segregation may occur as the granulation is transferred into other containers or while being fed into the tablet press. This "in process" segregation can result in non uniform tablets.

Wet granulation techniques typically use solvents such as ethanol or isopropanol which dissolve the dexamethasone into a spray solution which is sprayed onto the tablet granulation. The problem with this approach is largely the environmental impact of venting the spent solvents into the environment. Solvent recovery technology exists but is very expensive. Industry has been pressed to climinate solvents wherever possible due to the costs of solvent recovery.

WO 96/40078A describes dissolving dexamethasone and polyvinyl pryollidone in ethanol. The solution is added drop-wise to guar gum to form granules.

JP 11130663 describes suspension of biotin in hydroxy propyl cellulose or hydroxyl propyl methylcellulose and water. The suspension is dispersed using a wet granulation technique.

WO - A - 98/22095 describes a pharmaceutical composition comprising dexamethasone, hydroxypropylmethylcellulose and sugar spheres.

US - A - 3231468 describes a pharmaceutical composition of dexamethasone which is manufactured using a wet granulation process in a planetary mixer.

GB - A - 970440 describes a pharmaceutical composition of dexamethasone which is manufactured by dry blending and direct compression.

WO - A -- 98/35701 describes the manufacture of a formulation of dexamethasone in which dexamethasone is dry blended followed by wet granulation.

There is therefore a clear need for an improved process for preparing a pharmaceutical granulation of dexamethasone.

### Statements of Invention

According to the invention there is provided a process for manufacturing a formulation of dexamethasone comprising:-
preparing a dilute aqueous suspension of dexamethasone with starch;
spraying the dexamethasone solution onto tablet carrier ingredients in a fluidised bed dryer, to provide a dry granulation;
containing dexamethasone in an amount of from 0.02% to 2.0% w/w and from 0.5% to 5.0% w/w of starch.

In once embodiment of the invention the starch is pre-gelatinised starch.

Preferably weight ratio of the viscosity increasing excipient to the pharmaceutical compound is from 12:1 to 60:1, most preferably approximately 30: 1.

In one embodiment of the invention the viscosity increasing excipient is present in an amount of from 0.5% to 5.0% w/w of the tablet formulation.

In one embodiment the process comprises, prior to spraying, milling the aqueous mixture of dexamethasone and the viscosity increasing excipient to form a dexamethasone suspension.

The process may comprise suspending the starch in water and subsequently adding the dexamethasone to the suspension to form a mixture.

In one embodiment the resulting granulation is compressed into a tablet. Most preferably dexamethasone is present in the tablet in an amount of approximately 0.083% w/w.

The dexamethasone may be in the form of dexamethasone base.

The term granulation is taken throughout to include from very fine dusty powders to larger particle sizes such as granules.

The invention also provides a tablet formulation comprising and amount of from 2 to 0.02% w/w of the formulation of dexamethasone and from 0.5% to 5% w/w of the formulation of starch.

### Detailed description

The present invention utilizes a viscous aqueous based spray solution to provide a uniform distribution of a small amount of dexamethasone in a large amount of granulation. After the water evaporates, dexamethasone is bound to the granulation, reducing the potential for downstream segregation.

It was surprisingly found that dexamethasone could be combined with water and a viscosity building excipient and suspended in a spray solution.

Starch or pregelatinised starch is used to provide sufficient viscosity to the spray solution. The amount required depends on a number of factors such as the time a solution is left standing, and the degree of agitation of the mixer.

In the invention starch was used as the viscosity increasing excipient to make the spray solution comprising dexamethasone. A range of concentrations of starch paste were prepared and milled to observe the cut off point for the dexamethasone separating out. The range of 0.5% to 5.0% was investigated. The 0.5% sample showed some settling, but was readily dispersed. Even the 0.5% concentration would be acceptable when it is continuously mixed during spraying.

In the invention dexamethasone is incorporated into a very dilute paste with sufficient viscosity to prevent separation and ensure a uniform, suspended ingredient. The spray solution must be sufficiently dilute to be sprayed on the batch. The excipients providing viscosity to the aqueous solution act as "glue" and bind the dexamethasone to the granulation after the water evaporates away. The resulting granulation is uniform and the dexamethasone is bound to the granulation which minimizes segregation during subsequent product transfer.

Base ingredients may include any conventionally used carrier ingredient commonly used in the art. For example: lactose, microcrystalline cellulose, dibasic calcium phosphate, starch, dextrin, maltodextrin, compressible sugar, dextrose, and calcium sulfate. The mixing sequence used for dexamethasone includes preparing the starch past, adding dexamethasone to a portion of the starch paste to make a slurry (lumps may be present), then milling the slurry once or multiple times until it is a uniform suspension, followed by purging the mill with starch paste to remove residual dexamethasone in the mill. The milled suspension and purge are then added to the spray solution tank for spraying onto the granulation using a Glatt Fluid bed drier.

Advantageously, the starch is approximately 0.5% to 5.0% weight/weight of the batch.

The composition may be provided in the form of a uniform granulation which may be used a powder dosage form.

Alternatively the composition may be provided in the form of a uniform granulation which can be compressed into tablets.

The invention will be more clearly understood by the following example.

### Example

A 540 kg batch of dexamethasone 0.25 mg tablets was prepared according to the following procedure:

### Composition:

| Ingredient | Quantity |
|---|---|
| Dexamethasone | 0.45kg |
| Starch USP | 5.4kg |
| Water, Cold | 10.8 kg |
| Water, Hot | 178.2 kg |
| Granulation base ingredients | QS to 540kg |

### Procedure:

1. Add the starch to the cold water with stirring to make a smooth suspension.
2. Add the suspension (step 1) to the hot water with stirring. A starch paste will quickly form.
3. Remove approximately 18 kg of starch paste and place in a suitable container.
4. Add the dexamethasone to the container and mix together to get a dispersion, there may he lumps in the dispersion.
5. Pass the dispersion through a suitable mill to break up lumps of dexamethasone and produce a smooth uniform dispersion. In this example, the mill was a Charlotte Colloid Mill, Model # SD2.
6. Pass the dispersion through the mill again to further ensure the dexamethasone is all milled.
   (Repeat milling may be required to provide a uniform suspension).
7. Purge the mill with approximately 14 kg starch paste to remove residual dexamethasone.
8. Add the milled dexamethasone slurry and the purge back into the starch paste.
9. Mix until uniform in appearance.
10. Spray the dexamethasone suspension on the base ingredients in the a Glatt Fluid Bed Agglomerator / Drier, Model WSG ... 500.
11. Dry the granulation in the Glatt Drier to 2-3% moisture.
12. Remove the granulation from the Glatt drier and transfer to a Tote bin.
13. Assay the granulation and calculate tablet weight needed for 100% potency.
14. Using the target weight, compress the granulation into the tablets.

The resulting dexamethasone 0.25 mg tablets are uniform in potency. Content uniformity testing gave an average of 101.2% claim with an RSD of 0.8%.

The invention is not limited to the embodiments hereinbefore described which may be varied in detail.

## Claims

1. A process for manufacturing a formulation of dexamethasone comprising:-
preparing a dilute aqueous suspension of dexamethasone with starch;
providing in a fluidised bed dryer carrier ingredients used to form pharmaceutical granulations;
spraying the dexamethasone solution onto the carrier ingredients in the fluidised bed dryer, to provide a dry granulation, containing dexamethasone in an amount of from 0.02% to 2.0% w/w and from 0.5% to 5.0% w/w of starch.

2. A process as claimed in claim 1 wherein the starch is pre-gelatinised starch.

3. A process as claimed in claim 1 or 2 comprising, prior to spraying, milling the aqueous mixture of dexamethasone and starch to form a dexamethasone suspension.

4. A process as claimed in any of claims 1 to 3 comprising suspending the starch in water and subsequently adding the dexamethasone to the suspension to form a mixture.

5. A process as claimed in any receding claim wherein the granulation is in the form of a powder dosage form.

6. A process as claimed in any preceding claim wherein the granulation is compressed into a tablet dosage form.

7. A process as claimed in claim 5 or 6 wherein dexamethasone is present in the dosage form in an amount of approximatel 0.083% w/w.

8. A process as claimed in any of claims 1 to 7 wherein the dexamethasone is in the form of dexamethasone base.

## Patentansprüche

1. Verfahren zur Herstellung einer Dexamethason-Formulierung, umfassend:
Herstellen einer verdünnten, wässrigen Dexamethason-Suspension mit Stärke;
Bereitstellen von Trägerbestandteilen in einem Wirbelschichttrockner, die zur Bildung pharmazeutischer Granulate verwendet werden;
Sprühen der Dexamethason-Lösung auf die Trägerbestandteile im Wirbelschichttrockner, zum Bereitstellen eines Trockengranulats, das Dexamethason in einer Menge von 0,02 Gew.-% bis 2,0 Gew.-% und von 0,5 Gew.-% bis 5,0 Gew.-% Stärke enthält.

2. Verfahren nach Anspruch 1, worin die Stärke vorverkleisterte Stärke darstellt.

3. Verfahren nach Anspruch 1 oder 2, umfassend, vor dem Sprühen, das Vermahlen des wässrigen Gemischs aus Dexamethason und Stärke zum Bilden einer Dexamethason-Suspension.

4. Verfahren nach einem der Ansprüche 1 bis 3, umfassend das Suspendieren der Stärke in Wasser und im Anschluss daran Zufügen des Dexamethasons zur Suspension zum Bilden eines Gemischs.

5. Verfahren nach einem der vorangehenden Ansprüche, worin das Granulat in der Form einer Pulver-Dosierungsform vorliegt.

6. Verfahren nach einem der vorangehenden Ansprüche, worin das Granulat in eine Tabletten-Dosierungsform komprimiert wird.

7. Verfahren nach Anspruch 5 oder 6, worin Dexamethason in der Dosierungsform in einer Menge von ca. 0,083 Gew.-% vorliegt.

8. Verfahren nach einem der Ansprüche 1 bis 7, worin das Dexamethason in der Form einer Dexamethason-Base vorliegt.

## Revendications

1. Procédé de fabrication d'une formulation de déxaméthasone, comprenant :
la préparation d'une suspension aqueuse diluée de déxaméthasone avec de l'amidon ;
la fourniture, dans un séchoir à lit fluidisé, d'ingrédients véhicules utilisés afin de former des granulations pharmaceutiques ;
la pulvérisation de la solution de déxaméthasone sur les ingrédients véhicules dans le séchoir à lit fluidisé, afin de fournir une granulation sèche, contenant de la déxaméthasone en une quantité allant de 0,02% à 2,0% p/p et de 0,5% à 5,0% p/p d'amidon.

2. Procédé selon la revendication 1, dans lequel l'amidon est de l'amidon prégélatinisé.

3. Procédé selon la revendication 1 ou 2, comprenant, préalablement à la pulvérisation, le broyage du mélange aqueux de déxaméthasone et d'amidon afin de former une suspension de déxaméthasone.

4. Procédé selon l'une quelconque des revendications 1 à 3, comprenant la mise en suspension de l'amidon dans l'eau puis l'ajout de la déxaméthasone à la suspension afin de former un mélange.

5. Procédé selon l'une quelconque des revendications précédentes, dans lequel la granulation est sous la forme d'une forme de dosage pulvérulente.

6. Procédé selon l'une quelconque des revendications précédentes, dans lequel la granulation est comprimée sous forme de dosage en comprimés.

7. Procédé selon la revendication 5 ou 6, dans lequel la déxaméthasone est présente dans la forme de dosage en une quantité d'environ 0,083% p/p.

8. Procédé selon l'une quelconque des revendications 1 à 7, dans lequel la déxaméthasone est sous la forme d'une base de déxaméthasone.
